(19) Europäisches Patentamt
European Patent Office
Office européen des brevets

(11) Publication number: 0 265 247 B1

# (12) EUROPEAN PATENT SPECIFICATION

(45) Date of publication of the patent specification: **08.08.90**

(51) Int. Cl.[5]: **C07C 321/16**, C07C 317/00, A61K 31/10

(21) Application number: **87309315.7**

(22) Date of filing: **21.10.87**

(54) **Cyclopentyl ethers and their preparation and pharmaceutical formulation.**

(30) Priority: **22.10.86 GB 8625323**
**22.10.86 GB 8625324**

(43) Date of publication of application:
**27.04.88 Bulletin 88/17**

(45) Publication of the grant of the patent:
**08.08.90 Bulletin 90/32**

(84) Designated Contracting States:
**CH DE FR GB IT LI**

(56) References cited:
**EP-A- 0 013 107**
**DE-A- 2 256 537**
**DE-B- 2 902 699**
**US-A- 4 212 984**

(73) Proprietor: **GLAXO GROUP LIMITED, Clarges House 6-12 Clarges Street, London W1Y 8DH(GB)**

(72) Inventor: **Collington, Eric W., 28 Watton Road, Knebworth Hertfordshire(GB)**
Inventor: **Finch, Harry, 31 Newlands, Bletchworth Hertfordshire SG6 2JE(GB)**
Inventor: **Judd, Duncan B., 24 St. Margarets Road St. Margarets, Ware Hertfordshire(GB)**
Inventor: **Meadows, James D., 27D High Street Puckeridge, Ware Hertfordshire(GB)**

(74) Representative: **Skailes, Humphrey John et al, Frank B. Dehn & Co. Imperial House 15-19 Kingsway, London WC2B 6UZ(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

## Description

Prostaglandin $E_2$ is a naturally occurring substance which has many physiological actions. For example, it inhibits gastric acid secretion and provides gastrointestinal cytoprotection, lowers blood pressure, stimulates and relaxes smooth muscle, inhibits platelet aggregation and inhibits lipolysis.

Synthetic $PGE_2$ analogues offer the possibility of different potency, longer duration of activity and increased selectivity of action and are therefore of considerable interest.

We have now found a new group of cyclopentyl ethers that have $PGE_2$-type activity. Compounds in this class have a particularly useful profile of biological action. In particular they have shown high potency and improved selectivity as regards the inhibition of gastric acid secretion and gastrointestinal cytoprotection and are therefore of interest in the treatment of ulcers. Compounds according to the invention also have a lipid lowering action and are of interest in the treatment of clinical conditions in which the underlying aetiology is associated with lipid imbalance or hyperlipidemia.

The invention thus provides compounds of the general formula (1)

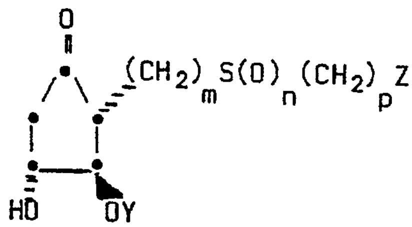

(1)

wherein m is 1 to 4
n is zero, 1 or 2;
p is 1 to 4;
Z is a group $CO_2R^1$ where $R^1$ is
(a) a hydrogen atom or a $C_{1-6}$ alkyl, $C_{7-10}$ phenalkyl or 2-naphthyl group;
(b) phenyl [optionally substituted by $C_{1-4}$ alkyl, $C_{1-4}$ alkoxy, $C_{1-4}$alkanoyl, methylthio, methylsulphinyl, methylsulphonyl, halogen, $-CO_2R^2$ (where $R^2$ is a hydrogen atom or $C_{1-4}$ alkyl or phenyl), $-NHCOR^2$ (where $R^2$ is as defined above or is a phenyl group optionally substituted by hydroxyl, $CH_3CONH$- or

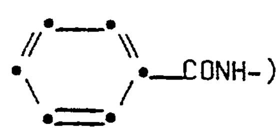

),

$-CONR^3R^4$ (where $R^3$ and $R^4$ may be the same or different and are each a hydrogen atom or a $C_{1-4}$ alkyl group), $-NHCONH_2$, $-CH_2CH(CONH_2)NHCOCH_3$ or

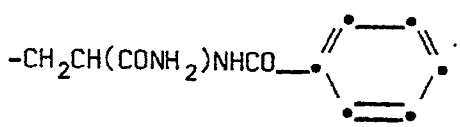

];

(c) a group $-CH_2COR^5$ where $R^5$ is phenyl [optionally substituted by $C_{1-4}$ alkyl, $C_{1-4}$ alkoxy, $C_{1-4}$ alkanoyl, methylthio, methylsulphinyl, methylsulphonyl, halogen, $-CO_2R^6$ (where $R^6$ is a hydrogen atom or $C_{1-4}$alkyl or phenyl, $-CONR^7R^8$ (where $R^7$ and $R^8$ may be the same or different and are each a hydrogen atom or a $C_{1-4}$ alkyl group), $-NHCOR^6$ (where $R^6$ is as defined above, or is a phenyl group optionally substituted by hydroxyl, $CH_3CONH$- or

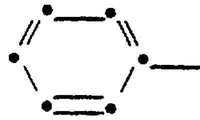 CONH-), $-NHCONH_2$, $-CH_2CH(CONH_2)NHCOCH_3$ or

$-CH_2CH(CONH_2)NHCO$ 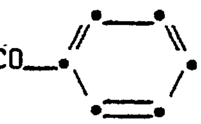 ]

or $R^5$ is 2-naphthyl;
or Z is $-CH_2OH$, -CHO or $CONHR^9$ [where $R^9$ is a hydrogen atom or $C_{1-4}$alkyl, aryl, $-COR^{10}$ (where $R^{10}$ is a hydrogen atom or a $C_{1-4}$ alkyl or aryl group) or $-SO_2R^{11}$ (where $R^{11}$ is a $C_{1-4}$ alkyl or aryl group)];
Y is

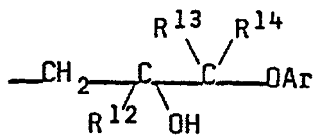

where $R^{12}$, $R^{13}$ and $R^{14}$ are each a hydrogen atom or methyl and at least one is a hydrogen atom, and Ar is a phenyl group (optionally substituted by one or two $C_{1-4}$ alkyl, $C_{1-4}$ alkoxy, $C_{1-4}$ alkylthio, $C_{1-4}$alkylsulphinyl, $C_{1-4}$ alkylsulphonyl, halogen or trifluoromethyl groups);
and the physiologically acceptable salts, solvates and complexes (e.g. cyclodextrin complexes) thereof.

The structural formula herein are to be understood to include the enantiomers of each of the compounds concerned as well as mixtures of the enantiomers including racemates.

In general, the compounds of formula (1) in which the carbon atom carrying the group $-(CH_2)_mS(O)_n(CH_2)_pZ$ and/or the carbon atom in the group Y carrying the -OH group are in the R-configuration (particularly the former) and mixtures containing such isomers are preferred.

The term 'alkyl' as a group or part of a group within the definition of the compounds of formula (1) is intended to cover straight or branched chain moieties, and may be, for example, a methyl, ethyl, n-propyl, i-propyl, n-butyl, s-butyl, or t-butyl group. The term 'halogen' means fluorine, chlorine, bromine, or iodine.

The aryl group referred to above in the definition of Z may be, for example, phenyl.

Compounds of formula (1) in which Z is $CO_2H$ or contains a $CO_2H$ grouping can form salts with bases. Examples of such salts are alkali metal (e.g. sodium), alkaline earth metal (e.g. calcium) and amine (e.g. piperazine) salts.

When Z is a group $CO_2R^1$, examples of suitable $R^1$ groups of type (a) are hydrogen, $C_{1-3}$ alkyl, benzyl, phenethyl and 2-naphthyl, and $R^1$ is preferably hydrogen or, more preferably, methyl or 2-naphthyl.

When Z is a group $CO_2R^1$, examples of suitable $R^1$ groups of type (b) are phenyl optionally substituted in the ortho, meta or, in particular, para position by a chlorine or bromine atom or a methyl, ethyl, propyl, n-butyl, t-butyl, methoxy, ethoxy, propoxy, butoxy, acetyl, propionyl, methylthio, methylsulphinyl, methylsulphonyl, $-CO_2H$, $-CO_2CH_3$, $-CO_2CH_2CH_3$,

$-CO_2$ 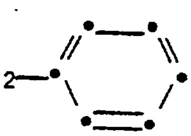 ,

-NHCHO, $-NHCOCH_3$, benzoylamino, (acetylamino)benzoylamino, (hydroxy)benzoylamino, $-CONH_2$, $-CONHCH_3$, $-CON(CH_3)_2$, $-CONHCH_2CH_3$, $-CON(CH_2CH_3)_2$, $-NHCONH_2$, $-CH_2CH(CONH_2)NHCOCH_3$ or

$-CH_2CH(CONH_2)NHCO$ 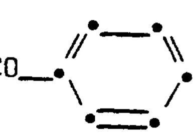 group.

Particularly useful substituents which may be present on the $R^1$ phenyl group include $C_{1-4}$alkoxy, $C_{1-4}$alkanoyl, methylthio, methylsulphonyl, $-CO_2R^2$, $-NHCOR_2$, $-CONR^3R^4$ [where $R^2$, $R^3$ and $R^4$ are as defined for formula (1)], $-NHCONH_2$, or $-CH_2CH(CONH_2)NHCOCH_3$. Especially useful substituents of this type include methoxy, acetyl, methylthio, methylsulphonyl, $-CO_2CH_3$, $-NHCOCH_3$, benzoylamino, (p-acetylamino)benzoylamino, (p-hydroxy)benzoylamino, $-CONH_2$, $-CON(CH_3)_2$, $-NHCONH_2$ or $-CH_2CH(CONH_2)NHCOCH_3$.

In general, when $R^1$ is a group of type (b) it is preferably a phenyl group substituted (particularly in the para position) by a methoxy, acetyl, $-CO_2CH_3$, $-NHCOCH_3$, benzoylamino, $-CONH_2$, $-CON(CH_3)_2$ or $-CH_2CH(CONH_2)NHCOCH_3$ group.

When Z is a group $CO_2R^1$, examples of suitable $R^1$ groups of type (c) are $-CH_2COR^5$ where $R^5$ is a phenyl group substituted (particularly in the para position) by a methoxy, acetyl, $-CO_2CH_3$, $-NHCOCH_3$, benzoylamino, $-CONH_2$, $-CON(CH_3)_2$ or $-CH_2CH(CONH_2)NHCOCH_3$ group, or $R^5$ is a 2-naphthyl group. Especially useful substituents of this type include $-NHCOCH_3$, benzoylamino, acetyl or $-CONH_2$ preferably attached at the para position on the phenyl group.

When Z is other than $CO_2R^1$ examples of suitable Z groups are $-CH_2OH$, $-CHO$ or $-CONHR^9$ [where $R^9$ is a hydrogen atom or methyl, ethyl, phenyl, $-COR^{10}$ (where $R^{10}$ is methyl or phenyl) or $-SO_2R^{11}$ (where $R^{11}$ is methyl or phenyl)], and is preferably $-CH_2OH$, $-CHO$, $-CONH_2$, $-CONHCH_3$, $-CONHCOCH_3$, $-CONHSO_2CH_3$ or

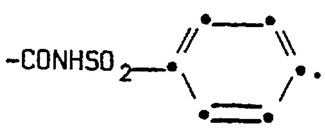

In the group Y, $R^{13}$ and $R^{14}$ are preferably hydrogen atoms.

When the Ar phenyl group is substituted, the substituent may for example be methyl, ethyl, propyl, butyl, methoxy, ethoxy, propoxy, butoxy, methylthio, methylsulphinyl, methylsulphonyl, fluoro, chloro, bromo or trifluoromethyl. Preferably, only a single substituent is present, particularly at the para-position. In general, Ar is preferably phenyl or phenyl substituted by halogen, particularly fluoro or chloro.

In general compounds of formula (1) in which n is zero are preferred.

The preferences indicated above apply both separately and in combination with one or more of the other stated preferences.

A preferred group of compounds of the invention thus has the formula (1) in which:

n is zero

m is 3 or 4 and p is 1 or

m is 2 or 3 and p is 2 or

m is 3 and p is 4 or

m is 2 and p is 3;

Z is a group $CO_2R^1$ [where $R^1$ is a hydrogen atom or $C_{1-3}$ alkyl (particularly methyl), 2-naphthyl or phenyl substituted (preferably in the para position) by methoxy, acetyl, $-CO_2CH_3$, $-NHCOCH_3$, benzoylamino, $-CONH_2$, $-CON(CH_3)_2$ or $-CH_2CH(CONH_2)NHCOCH_3$];

$R^{12}$ is a hydrogen atom or methyl;

$R^{13}$ and $R^{14}$ are hydrogen atoms;

Ar is phenyl or phenyl substituted by fluoro or chloro; including the physiologically acceptable salts, solvates and complexes (e.g. cyclodextrin complexes) thereof.

Compounds of this type in which the carbon atom carrying the $-(CH_2)_mS(O)_n(CH_2)_pZ$ group is in the R-configuration are particularly preferred. Especially preferred compounds of this type are those in which Z is a group $CO_2R^1$ where $R^1$ is a hydrogen atom or, more preferably, methyl or phenyl substituted in the para position by $-CO_2CH_3$ or benzoylamino.

Particularly important compounds of the invention are :

[1 R-[1α,2β( R*),3α]]-4-(Benzoylamino)phenyl 4-[[2-[3-hydroxy-2-(2-hydroxy-3-phenoxypropoxy)-5-oxocyclopentyl]ethyl]thio]butanoate; [1 R-[1α,2β( R*),3α]]-Methyl 4-[[[[2-[3-hydroxy-2-(2-hydroxy-3-phenoxypropoxy)-5-oxocyclopentyl]ethy ]thio]-l-oxobutyl]oxy]benzoate; and [1R-[1α,2β( R*),3α]]-(-) Methyl 4-[[2-[-3-hydroxy-2-(2-hydroxy-3-phenoxypropoxy)-5-oxocyclopentyl]ethyl]thio]butanoate; and complexes (e.g. cyclodextrin complexes) thereof.

Compounds of formula (1) inhibit gastric secretion, as determined for example by their ability to inhibit histamine-induced secretory responses in the rat perfused stomach, following the method of Ghosh and Schild in Br.J.Pharmacol., 1958, 13, 54 as modified by Parsons M.E., Ph.D Thesis, University of London, 1969.

The compounds also provide gastrointestinal cytoprotection, as determined for example by their ability to inhibit ethanol-induced lesions in the conscious rat, following the method of Robert et al in Gastroenterology, 1979, 77, 433, modified by the use of 5mg/kg/s.c. indomethacin prior to the administration of the test compound.

Compounds of the invention are also able to lower lipid levels as may be demonstrated in standard animal models for example by determining their ability to lower non-esterified fatty acid levels in the starved rat (P. P. Lovisolo et. al., PharmacologicalResearchCommunications, 1981, 13, 163-174; E. Schillinger and O. Loge, BiochemicalPharmacology, 1974, 23, 2283-2289).

The compounds are thus of interest in the prevention and/or treatment of ulcers. They may also be used in the treatment of other conditions which arise from the hypersecretion of gastric acid. They may also be used for the prevention and/or treatment of conditions in which the underlying aetiology is associated with lipid imbalance or hyperlipidemia.

According to a further aspect of the present invention we therefore provide a compound of formula (1) or a physiologically acceptable salt or complex (eg cyclodextrin complex) thereof for use in the prevention and/or treatment of ulcers and other conditions arising from hypersecretion of gastric acid. We also provide a compound of formula (1) or a physiologically acceptable salt or complex (e.g. cyclodextrin complex) thereof for use in the prevention and/or treatment of conditions in which the underlying aetiology is associated with lipid imbalance or hyperlipidemia.

According to another aspect of the invention we provide a method of treating the human or non-human animal body to combat ulcers and other conditions arising from hypersecretion of gastric acid or conditions in which the underlying aetiology is associated with lipid imbalance or hyperlipidemia, which method comprises administering to the said body an effective amount of a compound of formula (1) or a physiologically acceptable salt or complex (e.g. cyclodextrin complex) thereof.

It will be appreciated that the compounds according to the invention may advantageously be used in conjunction with one or more other therapeutic agents, such as non-steroidal anti-inflammatory agents, or different anti-ulcer agents. It is to be understood that the present invention covers the use of a compound of formula (1) or a physiologically acceptable salt or complex (eg cyclodextrin complex) thereof in combination with one or more other therapeutic agents.

In a further aspect of the present invention we provide a pharmaceutical composition comprising as an active ingredient a compound of formula (1) or a physiologically acceptable salt or complex (eg cyclodextrin complex) thereof together with one or more pharmaceutical carriers or excipients.

Compounds may be formulated in conventional manner with one or more pharmaceutical carriers, for example for oral, buccal, parenteral or rectal administration.

The compounds may be formulated for oral administration as, for example, tablets, capsules, powders, solutions or syrups prepared by conventional means with acceptable excipients.

The compounds may be formulated for parenteral administration by bolus injections or continuous infusion. Formulations for injections may be presented in unit dosage form in ampoules, or in multi-dose containers, with an added preservative.

For buccal administration, the compounds may be formulated as tablets or lozenges in conventional manner; and for rectal administration compositions such as suppositories or retention enemas, for example containing conventional suppository bases such as cocoa butter or other glyceride, can be used.

The compounds are preferably administered orally, for example in amounts of 0.5 to 300 µg/kg body weight, 1 to 4 times daily. For parenteral administration, the compounds may be administered in amounts of 0.01 to 10µg/kg body weight, 1 to 4 times daily. The precise dose will of course depend on the age and condition of the patient.

Suitable methods for preparing the compounds of the invention are described below, the various groups and symbols being as defined above except where otherwise indicated.

(a) Compounds of formula (1) may be prepared by deprotection of a compound of formula (2)

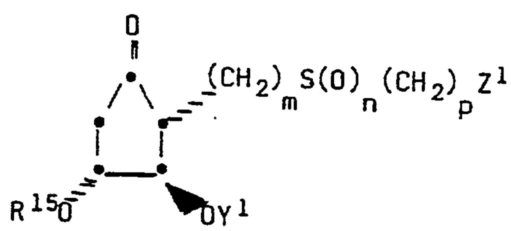

(2)

in which $Y^1$ is defined as a group

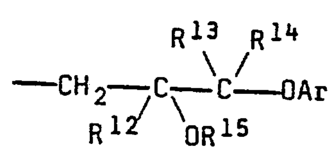

and $Z^1$ is Z as defined for formula (1) or is a group $-CH_2OR^{15}$ and $R^{15}$ is a suitable hydroxyl protecting group [e.g. tetrahydropyran-2-yl, tetrahydrofuran-2-yl, ethoxyethyl tri(hydrocarbyl)silyl or arylmethyl]

The $R^{15}$ groups in the compounds of formula (2) are conveniently the same, but they may be different if desired.

Where $R^{15}$ is tri(hydrocarbyl)silyl the hydrocarbyl substituents may be the same or different e.g. $C_{1-6}$ alkyl, $C_{2-6}$ alkenyl, $C_{3-7}$ cycloalkyl, $C_{7-20}$ aralkyl and $C_{6-20}$ aryl groups. Such groups include methyl, ethyl, n-propyl, isopropyl, n-butyl, isobutyl, t-butyl, allyl, phenyl and benzyl. Preferred hydrocarbyl groups are $C_{1-4}$ alkyl, e.g. methyl and t-butyl. Trimethylsilyl and t-butyldimethylsilyl groups are particularly preferred.

When $R^{15}$ is an arylmethyl group it may contain up to 20 carbon atoms, e.g. benzyl, diphenylmethyl or triphenylmethyl.

The method used to deprotect the protected hydroxyl group depends on the nature of $R^{15}$ but in general acid hydrolysis or reduction may be used.

Thus, for example when $R^{15}$ is a tetrahydropyran-2-yl, tetrahydrofuran-2-yl or ethoxyethyl group deprotection may be carried out with an acid. Suitable acids include inorganic acids such as hydrochloric acid and organic acids such as acetic acid or trifluoroacetic acid. Suitable solvents include ethers (e.g. diethyl ether, dioxan and tetrahydrofuran), halogenated hydrocarbons (e.g. dichloromethane), hydrocarbons (e.g. toluene), dipolar aprotic solvents (e.g. acetone, acetonitrile, dimethylsulphoxide and dimethylformamide) and alcohols (e.g. methanol, ethanol and ethylene glycol). Where desired the solvents may be used in combination with water. The reaction may be carried out at any suitable temperature, such as from 0° to 50°C, e.g. 40° to 50°C.

A tri(hydrocarbyl)silyl group may for example be removed by acid hydrolysis, e.g. with dilute mineral acid or trifluoroacetic acid or by treatment with fluoride ion (e.g. from a quaternary ammonium fluoride such as tetra-n-butyl ammonium fluoride), or by treatment with aqueous hydrogen fluoride. Arylmethyl groups may be removed by reduction, e.g. by hydrogenolysis, e.g. with a noble metal catalyst such as platinum or palladium, or by treatment with a Lewis acid (e.g. boron trifluoride-etherate) in the presence of a thiol (e.g. ethanethiol) in a suitable solvent such as dichloromethane at e.g. room temperature.

Compounds of formula (2) may be prepared by oxidation of a compound of formula (3)

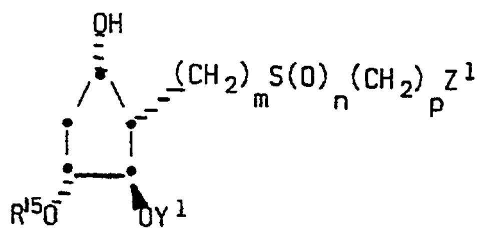

(3)

(where $Y^1$, $Z^1$ and $R^{15}$ are as defined just above) with for example dimethylsulphoxide, activated by N,N'-dicyclohexylcarbodiimide, in the presence of pyridinium trifluoroacetate in a solvent such as dichloromethane at e.g. -10°C to room temperature. Other conventional oxidative methods can also be used, for example pyridinium chlorochromate, pyridinium dichromate or Jones reagent.

In this reaction the $Z^1$ group is other than -CHO. When $Z^1$ is $-CH_2OH$, the -OH group will need to be protected in this reaction and may be for example a group $-CH_2OR^{15}$ described above.

It will be appreciated that the deprotection method (a) is usually applied in connection with the formation by oxidation of the cyclopentyl ring oxo group. Thus, the compounds of formula (1) may generally be prepared by oxidising a corresponding compound of formula (3) and the protecting groups removed thereafter.

The formation of the ring oxo group may however be effected prior to the introduction of the desired Z group by method (b), (c), (f) or (g) below) and the protecting group(s) removed thereafter.

Compounds of formula (2) in which $Z^1$ is -CHO may be prepared by oxidising a corresponding compound in which $Z^1$ is $-CH_2OH$ using for example an activated sulphur reagent e.g. a N-chlorosuccinimide-dimethylsulphide complex in a suitable solvent (e.g. toluene or dichloromethane) at temperatures of for example -25° to +25°C or pyridine-$SO_3$ complex in dimethylsulphoxide, preferably at 0°C to room temperature.

Intermediates of formula (3) in which n is zero may be prepared by reaction of sulphonates of formula (4)

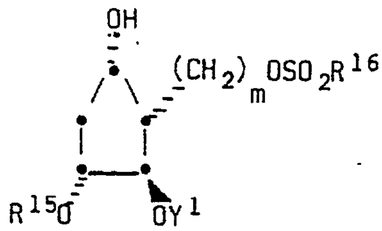

(4)

(in which $R^{15}$ and $Y^1$ are as defined above and $R^{16}$ is $C_{1-3}$ alkyl or aryl, e.g. monocyclic aryl such as 4-methylphenyl) with a thiol $HS(CH_2)_pZ^1$ or salts thereof (e.g. the sodium salt). The alkylation is preferably effected in the presence of base for example sodium hydride or potassium tert-butoxide in a solvent such as dimethylformamide at a suitable temperature (e.g. 0°C to room temperature).

The thiols $HS(CH_2)_pZ^1$ are known compounds or may be prepared using methods analogous to those used for the preparation of the known compounds.

Intermediates of formula (4) may be prepared by reacting a corresponding diol of formula (5)

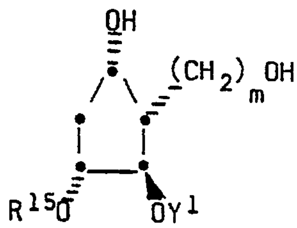

(5)

(in which $Y^1$ and $R^{15}$ are as defined above) with a sulphonyl halide, for example a sulphonyl chloride, $R^{16}SO_2Cl$ (where $R^{16}$ is as defined above). The sulphonylation is preferably effected in the presence of a suitable acid scavenger for example an organic base such as pyridine which can also be used as solvent. The reaction is preferably carried out at 0°C.

Although compounds of formula (4) can be isolated and purified they can also be prepared in situ and used immediately.

Compounds of formula (3) in which $Z^1$ is $-CO_2H$ may also be prepared by hydrolyzing a corresponding ester (e.g. a $C_{1-6}$ alkyl ester) e.g. using a base such as sodium hydroxide or potassium hydroxide in a suitable solvent (e.g. methanol) at room temperature to 50°C.

Intermediate diols of formula (5) may be prepared as shown in the following sequences:

(i)

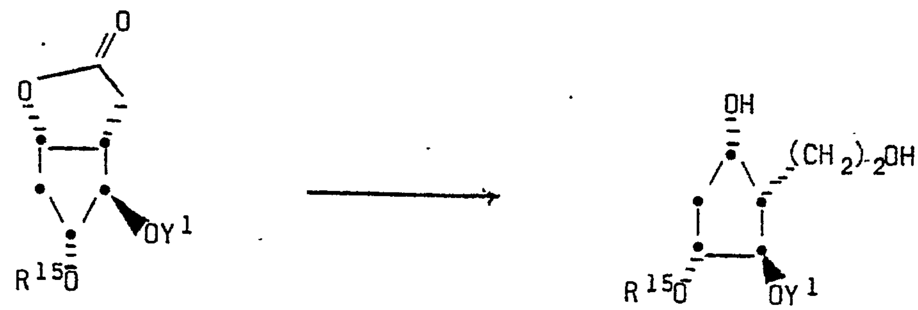

(6)

(5), m is 2

(ii)

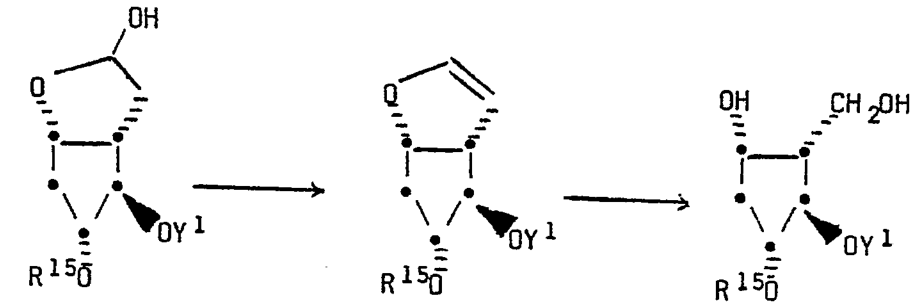

(7) (8) (5), m is 1

(iii)

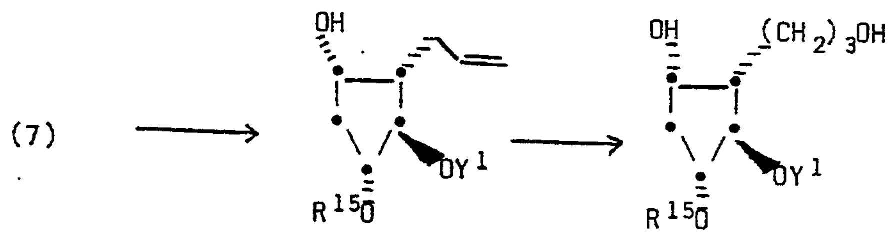

(9) (5), m is 3

(iv)

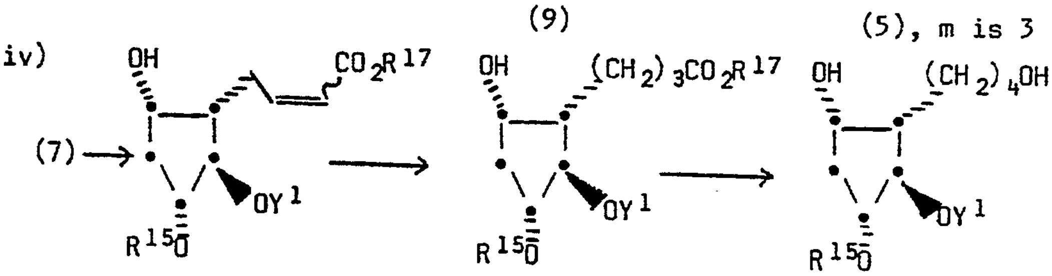

(10) (11) (5), m is 4

Thus, in sequence (i) diols of formula (5) in which m is 2 may be prepared by reduction of lactones of formula (6) with for example lithium aluminium hydride, sodium bis(2-methoxyethoxy)aluminium hydride or diisobutylaluminium hydride in a suitable solvent (e.g. tetrahydrofuran) at a suitable temperature (e.g. 0°C to room temperature).

In sequence (ii) diols of formula (5) in which m is 1 may be prepared by ozonolysis of an enol ether of formula (8) in a suitable solvent (e.g. an ethanol-dichloromethane mixture) at an appropriate temperature (e.g. -78°C), followed by an in situ reduction with for example sodium borohydride. The enol ethers of formula (8) may be prepared from a lactol formula (7) by reaction with a sulphonyl chloride $R^{16}SO_2Cl$ in the presence of a base (e.g. triethylamine or pyridine) in a suitable solvent (e.g. dichloromethane) at an appropriate temperature (e.g. -78°C to room temperature).

In sequence (iii), diols of formula (5) in which m is 3 may be prepared by treating an olefin of formula (9) with diborane and reacting the resulting intermediate with alkaline hydrogen peroxide. The reaction can be carried out in a suitable solvent (e.g. tetrahydrofuran) at a suitable temperature (e.g. 0°C to room

temperature). Olefins of formula (9) may be prepared by reacting a lactol of formula (7) with an appropriate Wittig reagent $(R^{18})_3P{=}CH_2$ (where $R^{18}$ is aryl e.g. monocyclic aryl such as phenyl). Suitable solvents include tetrahydrofuran and dimethylsulphoxide. The reaction may be carried out at any suitable temperature from -70°C to 50°C, preferably at room temperature.

In sequence (iv), diols of formula (5) in which m is 4 may be prepared by reduction of an ester of formula (11) by methods similar to those described above for the conversion of lactones of formula (6) into diols of formula (5) where m is 2. Esters of formula (11) can be prepared by reduction of the corresponding α,β-unsaturated esters of formula (10) e.g. by hydrogenation in the presence of a noble metal catalyst such as platinum or palladium, in a suitable solvent e.g. an alcohol (e.g. ethanol). The unsaturated esters of formula (10) may be prepared by reaction of lactols of formula (7) with an appropriate Wittig reagent $(R^{18})_3P{=}CHCO_2R^{17}$ (where $R^{17}$ is $C_{1-3}$ alkyl or $C_{7-10}$phenalkyl and $R^{18}$ is as defined above) in a suitable solvent for example ethanol, in the presence of an organic acid (e.g. acetic acid).

Lactones of formula (6) and lactols of formula (7) may be prepared by the methods described in European Patent Specification No. 160495.

(b) Compounds of formula (1) in which Z is $-CO_2R^1$ where $R^1$ is a group of type (a) or (b) may also be prepared by esterifying the corresponding compound in which Z is $-CO_2H$.

Such compounds may be prepared by conversion of the corresponding carboxylic acid into an activated derivative (e.g. a corresponding mixed anhydride) formed for example by reaction with an alkyl chloroformate (e.g. isobutyl chloroformate) or an acid chloride (e.g. pivaloyl chloride) in the presence of a suitable base (e.g. triethylamine or pyridine). The activated derivative can then be reacted with an appropriate alcohol, $R^1OH$, for example using a solvent such as dipolar aprotic solvent (e.g. acetone, acetonitrile or dimethylformamide) or a halogenated hydrocarbon (e.g. dichloromethane) at any suitable temperature (e.g. from -10°C to room temperature).

In addition, such compounds in which $R^1$ is a $C_{1-6}$ alkyl group may be prepared by esterification of the corresponding carboxylic acid with a diazoalkane e.g. diazomethane, in a suitable solvent (e.g. dichloromethane or ether) at room temperature.

(c) Compounds of formula (1) in which Z is $-CONHR^9$ may also be prepared by amidation of the parent carboxylic acids or alkyl esters of formula (1), i.e. the corresponding compounds of formula (1) in which Z is $-CO_2R^1$ (where $R^1$ is a hydrogen atom or a $C_{1-6}$alkyl group).

Conventional methods for converting acids and esters into amides may be used. For example, a reactive derivative of the carboxylic acid may be treated with a compound $R^9NH_2$ in a suitable solvent, e.g. acetone or acetonitrile. The reactive derivative is conveniently a mixed anhydride of the acid, formed for example by treatment of the acid with a chloroformate in the presence of a suitable base, e.g. triethylamine or pyridine. The chloroformate may for example be a $C_{1-6}$alkyl (e.g. isobutyl), aryl (e.g. phenyl) or aralkyl (e.g. benzyl) chloroformate. Alternatively, the reactive derivative may be an imidazolide, formed for example by treatment of the acid with 1,1′-carbonyldiimidazole.

The ring hydroxy group may need to be in a protected form during these reactions. Conventional methods of protection and deprotection may be used, as described above in process (a).

(d) Compounds of formula (1) in which n is 1 may also be prepared by oxidation of compounds of formula (1) in which n is zero with a suitable oxidizing agent, such as sodium periodate in a suitable solvent (e.g. methanol).

(e) Compounds of formula (1) in which n is 2 may also be prepared by oxidation of compounds of formula (1) in which n is zero or 1 with a suitable oxidising agent, such as a peracid (e.g. meta-chloroperoxybenzoic acid) in a suitable solvent (e.g. dichloromethane).

(f) Compounds of formula (1) in which Z is $-CO_2H$ may also be prepared by hydrolysis of a corresponding ester. The ester is desirably a labile ester, for example a compound of formula (1) in which Z is $-CO_2R^1$ where $R^1$ is tetrahydropyran-2-yl or tri(hydrocarbyl)silyl. Hydrolysis may be effected with an acid (e.g. acetic acid or trifluoroacetic acid) in a suitable solvent (e.g. aqueous tetrahydrofuran or dichloromethane) at an appropriate temperature.

(g) Compounds of formula (1) in which Z is $-CO_2R^1$ where $R^1$ is a group of type (c) may also be prepared by alkylation of the corresponding compound in which Z is $-CO_2H$ with a ketone of formula (12)

$XCH_2COR^5$(12)

[in which X is a leaving group such as halogen (e.g. bromine)]. The reaction is preferably carried out in the presence of a base such as diisopropylethylamine or potassium fluoride in a solvent such as acetonitrile or dimethylformamide at a suitable temperature (e.g. room temperature).

Ketones of formula (12) are known compounds or may be prepared by methods analogous to those used for the preparation of the known compounds.

(h) Where salts of compounds of formula (1) are desired such salts may be formed by conventional methods e.g. by treating an acid of formula (1) with a base (e.g. an amine such as piperazine) in a solvent such as ether.

Complexes (e.g. cyclodextrin complexes) may be prepared using conventional methods e.g. by treating a compound of formula (1) with α, β-, γ-cyclodextrin in a suitable solvent.

The processes in methods (b)-(g) may also be applied to compounds of formula (2) and in particular to compounds of formula (3) and the products subsequently converted into compounds of formula (1) by the methods described above.

When a specific enantiomer of formula (1) is required, starting materials having the desired stereochemical configuration should be used in the above processes. Such starting materials may be prepared for example using the methods described in European Patent Specification 160495 from an enantiomeric intermediate as described in European Patent Specification 74856.

The following examples illustrate the invention.

Temperatures are in °C.

'Dried' refers to drying with anydrous $MgSO_4$.

T.l.c. - Thin layer chromatography on silica.

Chromatography was carried out on silica gel.

The following abbreviations are used :

ER-ether; EA-ethyl acetate; PE-petroleum ether (b.p. 40-60° unless otherwise stated); THF-tetrahydrofuran; $CH_2Cl_2$-dichloromethane; $CHBr_3$-bromoform; DMSO-diemthylsulphoxide; MeOH-methanol; EtOH-ethanol; $Et_3N$-triethylamine; $LiAlH_4$-lithium aluminium hydride; HCl-hydrochloric acid; $NaHCO_3$-sodium bicarbonate; NaOH-sodium hydroxide; $CHCl_3$-chloroform.

Intermediate 1

[3aR-[3aα, 4α(2R*), 5β, 6aα]]-(+)-Hexahydro-4-[3-phenoxy-2-[tetrahydro-2H-pyran-2-yl)oxy] propox ]-5-[(tetrahydro-2H-pyran-2-yl)-oxy]-2H-cyclopenta[b]fu ran-2-one

Intermediate 2

[3aR-[3aα, 4α(2R*), 5β, 6aα]]-Hexahydro-4-[3-phenoxy-2-[(tetrahydro-2H-pyran-2-yl)oxy]propoxy]--[(tetrahydro-2H-pyran-2-yl)oxy]-2H-cyclopenta[b]furan- 2-ol

Intermediates 1 and 2 were prepared as described in European Patent Specification No. 160495.

Intermediate 3

[1S-[1α, 2β(2S*), 3α, 5α]]-5-Hydroxy-2-[3-phenoxy-2-[(tetrahydro-2H-pyran-2-yl)oxy] propoxy]-3 (tetrahydro-2H-pyran-2-yl)cyclopentan-ethanol

A solution of Intermediate 1 (1g) in THF (20mℓ) was added to a cooled, stirred mixture of $LiAlH_4$ (0.08g) in THF (5mℓ) under nitrogen. After 3h, further $LiAlH_4$ (0.08g) was added. After 2h, water (2mℓ) was added dropwise, the mixture was stirred for 10 min. and then partitioned between ER (50mℓ) and 2N HCl (50mℓ). The separated organic phase was washed with 8% $NaHCO_3$ solution (50mℓ) and brine and aqueous washings back-extracted with ER (50mℓ). The organic phases were dried and evaporated. The residue was purified by chromatography using EA→ EA-MeOH (97:3) as eluant to give the titlecompound as an oil (0.79g). I.r. ($CHBr_3$) 3600 and 3420cm$^{-1}$.

Intermediate 4

[1S-[1α, 2β(2S*), 3α, 5α]]-Methyl 4-[[2-[5-hydroxy-2-[3-phenoxy-2-[(tetrahydro-2H-pyran -2-yl)oxy]propoxy] 3-[(tetrahydro-2H-pyran-2-yl)oxy]cyclopentyl]ethyl]thio ]butanoate

4-Methylbenzenesulphonyl chloride (0.36g) and Intermediate 3 (0.69g) were dissolved in dry pyridine (6mℓ) at 0° under nitrogen. After 6h, a solution of γ-thiobutyrolactone (0.6mℓ) and sodium methoxide (0.27g) in MeOH (7mℓ), which had been pre-stirred under nitrogen for 1h, was added. After 18h at 0° the reaction mixture was allowed to warm to room temperature. After a futher 7h, ER (40mℓ) was added and the solution washed with 2N HCl (2 x 50mℓ), 8% aqueous $NaHCO_3$ (50mℓ) and saturated brine (50mℓ). The washings were back-extracted with ER and the combined organic layers dried. Solvent evaporation and purification by chromatography using ER-PE (9:1) as eluant gave the titlecompound as an oil (0.39g). I.r. ($CHBr_3$) 3580, 3510, 1725cm$^{-1}$.

Intermediate 5

[3aR-[3aα, 4α(2R*), 5β, 6aα]]-4, 5, 6, 6a-Tetrahydro-4-[3-phenoxy-2-[(tetrahydro-2H-pyran-2-yl)oxy]propoxy]-5- tetrahydro-2H-pyran-2-yl)oxy]-3aH-cyclopenta[b]furan

Methanesulphonyl chloride (1.0mℓ) was added to a stirred solution of Intermediate 2 (4.62g) in pyridine (30mℓ). After 18h the solution was cooled to -30° and water (0.4mℓ) was added. The solution was allowed to warm to room temperature and after 20 min., partitioned between ether (200mℓ) and 2N NaOH solution

(200mℓ), saturated brine (200mℓ), dried and then evaporated. The residue was purified by chromatography using ER as eluant to give the titlecompound as an oil (0.66g). T.l.c. (PE-ER, 3:2) Rf 0.24.

Intermediate 6

[1S-[1α, 2β(2S*), 3α, 5α]]-5-Hydroxy-2-[3-phenoxy-2-[tetrahydro-2H-pyran-2-yl)oxy]propoxy]-3-(tetrahydro-2H-pyran-2-yl)oxy]cyclopentane-methanol

Ozone was bubbled through a solution of Intermediate 5 (0.71g) in $CH_2Cl_2$ (30mℓ) and EtOH (12mℓ) containing Sudan III (0.5mg) at -78°. When the solution decolourised it was purged with nitrogen for 30min and then sodium borohydride (0.34g) was added. After 10 min. at -78°, 30 min. at 0° and 1h at room temperature, saturated ammonium chloride solution (50mℓ) was added. The mixture was extracted with $CH_2Cl_2$ (2 x 50mℓ) and the organic phases dried and evaporated. Purification of the residue by chromatography using EA-MeOH (98.5:1.5→ 96:4) as eluant gave the titlecompound as an oil (0.63g). I.r. (Neat) 3400cm$^{-1}$.

Intermediate 7

[1S-[1α, 2β, 3β(2S*), 4α]]-(+)-3-[3-Phenoxy-2-[(tetrahydro-2H-pyran-2-yl)oxy]propoxy]-2-(2-propenyl)-4-[(tetrahydro-2H-pyran-2-yl)oxy]cyclopentanol

Potassium t-butoxide (1.40g) and (methyl)triphenylphosphonium bromide (4.50g) were stirred in THF (50mℓ) for 20 min. under nitrogen. A solution of Intermediate 2 (2.09g) in THF (25mℓ) was added and after stirring at room temperature for 18h, saturated ammonium chloride solution (100mℓ) was added. Extraction with ER (2 x 100mℓ), drying, solvent evaporation and purification by chromatography using ER-PE (3:1) as eluant gave the titlecompound as an oil (1.98g). I.r. ($CHBr_3$) 3590, 3530cm$^{-1}$. $[\alpha]_D^{20}$ + 23.8$^0$ ($CHC\ell_3$).

Intermediate 8

[1S-[1α, 2β(2S*), 3α, 5α]]-(+)-5-Hydroxy-2-[3-phenoxy-2-[(tetrahydro-2H-pyran-2-yl)oxy]propox ]-3-(tetrahydro-2H-pyran-2-yl)oxy]-cyclopentane propanol

A solution of borane (1.0M is THF, 4.0mℓ) was added to a solution of Intermediate 7 (1.93g) in THF (30mℓ) at 0° under nitrogen. After 4h further borane solution (5.0mℓ) was added. After a further 2h water (5mℓ) was added, followed by 2N NaOH (4.5mℓ) and 30% aqueous hydrogen peroxide (3.4mℓ). After 30 min. 20% aqueous sodium sulphite (50mℓ) was added and after stirring for a further 30 min., the mixture was extracted with ER (2 x 60mℓ), the organic phases dried and then evaporated. Purification of the residue by chromatography using EA→ EA-MeOH (93:3) as eluant gave the titlecompound as an oil (1.84g). I.r. ($CHBr_3$) 3600, 3520cm$^{-1}$. $[\alpha]_D^{20}$ + 9.5$^0$ (MeOH).

Intermediate 9

[1S-[1α, 2β(2S*), 3α, 5α]]-Ethyl 4-[5-hydroxy-2-[3-phenoxy-2-[(tetrahydro-2H-pyran-2-yl)oxy]propoxy]-3-[ tetrahydro-2H-pyran-2-yl)-oxy]cyclopentyl]-2-butenoate. E:Z, 60:40

( Carbethoxymethylene)triphenylphosphorane (1.05g) was added to a stirred solution of Intermediate 2 (1.10g) in EtOH (40mℓ) containing one drop of acetic acid. After 5 days further (carbethoxymethylene)triphenylphosphorane (0.5g) was added. After a further 2 days solvent was evaporated and the residue partitioned between EA (40mℓ) and 8% aqueous $NaHCO_3$ (40mℓ). The organic layer was washed with saturated brine (40mℓ) and the aqueous layers back-extracted with EA (40mℓ). The combined organic layers were dried, solvent was evaporated and the residue purified by chromatography using ER-PE (9:1) as eluant to give the titlecompound as an oil (1.14g). I.r. ($CHBr_3$) 3520, 1705cm$^{-1}$.

Intermediate 10

[1S-[1α, 2β(2S*), 3α, 5α]]-(+)-Ethyl 4-[5-hydroxy-2-[3-phenoxy-2-[(tetrahydro-2H-pyran-2-yl)oxy]propoxy]-3-[ tetrahydro-2H-pyran-2-yl)oxy]cyclopentyl]butanoate

A suspension of 10% palladium oxide on charcoal (0.28g) in a solution of Intermediate 9 (1.08g) in EA (50mℓ) was stirred under an atmosphere of hydrogen for 2h. Filtration and solvent evaporation gave the titlecompound as an oil (1.06g). I.r. ($CHBr_3$) 3590, 3520, 1720cm$^{-1}$. $[\alpha]_D^{20}$ + 23.2° ($CHCl_3$).

Intermediate 11

[1S-[1α, 2β(2S*), 3α, 5α]]-(+)-5-Hydroxy-2-[3-phenoxy-2-[(tetrahydro-2H-pyran-2yl)oxy]propoxy -3-[(tetrahydro-2H-pyran-2-yl)oxy]cyclopentanebutanol

Sodium bis(2-methoxyethoxy)aluminium hydride (3.4M in toluene, 1.0mℓ) was added to a solution of Intermediate 10 (1.02g) in THF (20mℓ) at 0°C. After 1h Saturated ammonium chloride (25mℓ) was added and the mixture extracted with EA (2 x 40mℓ). Drying and solvent evaporation gave a residue which was purified by chromatography using EA as eluant to give the titlecompound as an oil (0.78g). I.r. ($CHBr_3$) 3600, 3520cm$^{-1}$. $[\alpha]_D^{20}$ + 26° ($CHCl_3$).

Intermediate 12

12a) [1S-[1α, 2β(2S*), 3α, 5α]]-5-Hydroxy-2-[3-phenoxy-2-[(tetrahydro-2H-pyran-2-yl)oxy]propoxy]-3 [(tetrahydro-2H-pyran-2-yl)oxy] cyclopentaneethanol 4-methylbenzenesulphonate

4-Methylbenzenesulphonyl chloride (0.47g) and Intermediate 3 (0.0g) were dissolved in pyridine (10mℓ) at 0° and the solution stirred for 8h to give the titlecompound. T.l.c. (ER-PE, 5:1) Rf 0.35.

12b) [1S-[1α, 2β(2S*), 3α, 5α]]-5-Hydroxy-2-[3-phenoxy-2-[(tetrahydro-2H-pyran-2-yl)oxy]propoxy]-3 [(tetrahydro-2H-pyran-2-yl)oxy]cyclopentanemethanol 4-methylbenzenesulphonate

A solution of Intermediate 6 (0.29g) and 4-methylbenzenesulphonyl chloride (0.15g) in pyridine (3mℓ) was kept at 4° for 24h. ER (40mℓ) was added and the solution washed with 2N HCl (40mℓ), 8% aqueous $NaHCO_3$ (40mℓ) and saturated brine (40mℓ). The washings were back-extracted with ER (30mℓ), the combined organic layers dried, and the solvent evaporated. The residue was purified by chromotography using ER-PE (9:1) as eluant to give the titlecompound as an oil (0.28g). I.r. ($CHBr_3$) 3580, 3520, 1360cm$^{-1}$.

The following compounds were prepared in a similar manner :

12c) [1S-[1α, 2β(2S*), 3α, 5α]]-5-Hydroxy-2-[3-phenoxy-2-[(tetrahydro-2H-pyran-2-yl)oxy]propoxy]-3 [(tetrahydro-2H-pyran-2-yl)oxy]cyclopentanepropanol 4-methylbenzenesulphonate. I.r. ($CHBr_3$) 3520, 1352cm$^{-1}$

From Intermediate 8.

12d) [1S-[1α, 2β(2S*), 3α, 5α]]-(+)-5-Hydroxy-2-[3-phenoxy-2-[(tetrahydro-2H-pyran-2-yl)oxy]propox] -3-[(tetrahydro-2H-pyran-2-yl)oxy]cyclopentanebutanol 4-methylbenzenesulphonate.
I.r. ($CHBr_3$) 3600, 3530, 1355cm $^{-1}$. $[\alpha]_D^{20}$ + 21° ($CHCl_3$).

From Intermediate 11.

Intermediate 13

13a) [1S-1α, 2β(2S*), 3α, 5α]]-Methyl-3-[[2-[5-hydroxy-2-[3-phenoxy-2-[(tetrahydro-2H-pyran-2-yl) xy]propoxy]-3-(tetrahydro-2H-pyran-2-yl)oxy]cyclopentyl ]ethyl]thio]propionate

A solution of methyl 3-mercaptopropionate (1.1mℓ) and potassium tert-butoxide (1.0g) in dimethylformamide (15mℓ) which had been pre-stirred for 1h was added to a solution of freshly prepared Intermediate 12a in pyridine (10mℓ) at 0°. After 17h at 0° ER (100mℓ) was added and the solution was washed with 2N HCl (100mℓ), 8% aqueous $NaHCO_3$ (100mℓ), water (100mℓ) and saturated brine (100mℓ). The washings were back-extracted with ER (100mℓ) and the combined organic layers dried. Evaporation in the presence of $Et_3N$ (1mℓ) gave a residue which was purified by chromatography using ER-PE (9:1) as eluant to give the titlecompound as an oil (0.45g). T.l.c. (ER-PE, 9:1) Rf 0.25.

13b) [1S-[1α, 2β(2S*), 3α, 5α]]-Methyl 5-[[[5-hydroxy-2-[3-phenoxy-2-[(tetrahydro-2H-pyran-2-yl)oxy]propoxy]-3 [(tetrahydro-2H-pyran-2-yl)oxy]cyclopentyl]methyl]thio] pentanoate

Sodium methoxide (0.22g) was added to a stirred solution of methyl 5-(acetylthio)pentanoate (0.8g) in dry MeOH (6mℓ) under nitrogen. After 3h the solvent was evaporated, the solid residue dissolved in dimethylformamide (5mℓ) and a solution of the Intermediate 12b (0.26g) in dimethylformamide (5mℓ) was added. After stirring at room temperature for 3 days, ER (70mℓ) was added and the solution washed with 2N HCl (70mℓ), water (3 x 70mℓ) and saturated brine (70mℓ). The aqueous washings were back-extracted with ER (70mℓ) and the combined organic layers dried. Solvent evaporation and purification by chromatography using ER-PE (9:1) as eluant gave the titlecompound as an oil (0.2g). I.r. ($CHBr_3$) 3490, 3430, 1731cm$^{-1}$.

The following compounds were prepared in a similar manner :

13c) [1S-[1α, 2β(2S*), 3α, 5α]]-Methyl [[3-[5-hydroxy-2-[3-phenoxy-2-[(tetrahydro-2H-pyran-2-yl)oxy]propoxy]-3 [(tetrahydro-2H-pyran-2-yl)oxy]cyclopentyl]propyl]thio] acetate.
I.r. ($CHBr_3$) 3520, 1728cm$^{-1}$.

From Intermediate 12c and methyl mercaptoacetate.

13d) [1S-[1α, 2β(2S*), 3α, 5α]]-Methyl 3-[[3-[5-hydroxy-2-[3-phenoxy-2-[(tetrahydro-2H-pyran-2-yl)oxy]propoxy] 3-[(tetrahydro-2H-pyran-2-yl)oxy]cyclopentyl]propyl]thi o]propionate.
T.l.c. (ER) Rf 0.34.

From Intermediate 12c and methyl 3-mercaptopropionate.

13e) [1S-[1α, 2β(2S*), 3α, 5α]]-(+) Methyl [[4-[5-hydroxy-2-[3-phenoxy-2-[(tetrahydro-2H-pyran-2-yl)oxy]propoxy]-3 [(tetrahydro-2H-pyran-2-yl)oxy]cyclopentyl]butyl]thio]a cetate.
I.r. ($CHBr_3$) 3520, 1728cm$^{-1}$. $[\alpha]_D^{20}$ + 25.6 ($CHCl_3$).

From Intermediate 12d and methyl mercaptoacetate.

Intermediate 14

[1S-[1α, 2β(2S*), 3α, 5α]]-4-[[2-[5-Hydroxy-2-[3-phenoxy-2-[(tetrahydro-2H-pyran-2-yl)oxy]pro oxy]-3-[(tetrahydro-2H-pyran-2-yl)oxy]cyclopentyl]ethyl ]thio]butanoic acid

5N NaOH (3mℓ) was added to a solution of Intermediate 4 (0.6g) in MeOH (14mℓ). After 2h the mixture was poured into water (100mℓ) and washed with ER (100mℓ). The aqueous extract was acidified with ammonium chloride (80mℓ) and extracted with EA (3 x 75mℓ). The combined organic extracts were dried and evaporated to leave the titlecompound as an oil (0.6g). I.r. ($CHBr_3$) 3500, 3400 - 2500, 1728cm$^{-1}$.

Intermediate 15

15a) [1S-[1α, 2β(2S*), 3α, 5α]]-4-(Benzoylamino)phenyl 4-[[2-[5-hydroxy-2-[3-phenoxy-2-[(tetrahydro-2H-pyran-2-yl)oxy]propoxy] 3-(tetrahydro-2H-pyran-2-yl)oxy]cyclopentyl]ethyl]thio] butanoate

Pivaloyl chloride (0.15mℓ) was added to a solution of Intermediate 14 (0.6g) and $Et_3N$ (0.5mℓ) in dry dimethylformamide (5mℓ) at 0°. After 15 min a solution of 4-(benzoylamino)phenol (0.53g) in dimethylformamide (2mℓ) was added and stirring continued for 6h at 0° and 18h at room temperature. The reaction mixture was diluted with EA (100mℓ) and washed with water (2 x 60mℓ) and brine (50mℓ). The aqueous washings were back-extracted with EA (100mℓ) and the combined organic phases dried and evaporated. Purification by chromatography on $Et_3N$ -deactivated silica gel using cyclohexane - EA (2:1) as eluant gave the titlecompound as a gum (0.35g). I.r. ($CHBr_3$) 3520, 3420, 1748, 1728, 1672 cm$^{-1}$.

The following compound was prepared in a similar manner:

15b) [1S-[1α, 2β(2S*), 3α, 5α]]-Methyl 4-[4-[[2-[5-hydroxy-2-[3-phenoxy-2-[(tetrahydro-2H-pyran-2-yl)oxy]propo y]-3-(tetrahydro-2H-pyran-2-yl)oxy]cyclopentyl]ethyl]th io]-1-oxobutoxy]benzoate.
I.r. ($CHBr_3$) 3520, 1754, 1715cm$^{-1}$.

From Intermediate 14 and 4-hydroxybenzoic acid methylester.

Intermediate 16

16a) [1R-[1α, 2β(2R*), 3α]]-4-(Benzoylamino)phenyl [4-[[2-[5-oxo-2-[3-phenoxy-2-[(tetrahydro-2H-pyran-2-yl)oxy]propoxy]-3- (tetrahydro-2H-pyran-2-yl)oxy]cyclopentyl]ethyl]thio]bu tanoate

A stirred solution of Intermediate 15a (0.33g) in dry $CH_2Cl_2$ (10mℓ) and dry DMSO (0.85mℓ) was treated with dicyclohexylcarbodiimide (0.53g) followed by pyridinium trifluoroacetate (0.33g). After 22h water (5mℓ) was added and after 5 min the mixture filtered through 'hyflo' and evaporated. The residue was dissolved in ER (20mℓ) and washed consecutively with water (10mℓ), 10% copper sulphate solution (20mℓ) and brine (20mℓ). The aqueous washings were back-extracted with ER (20mℓ) and the combined extracts dried and evaporated. Purification by chromatography on acid-washed (pH 3.8) silica using cyclohexane:EA (2:1) as eluant gave the titlecompound as an oil (0.25g). I.r. ($CHBr_3$) 3420, 1738, 1670cm$^{-1}$.

The following compounds were prepared in a similar manner:

16b) [1R-[1α, 2β(2R*), 3α]]-Methyl 4-[1-oxo-4-[[2-[5-oxo-2-[3-phenoxy-2-[(tetrahydro-2H-pyran-2-yl)oxy]pro oxy]-3-(tetrahydro-2H-pyran-2-yl)oxy]cyclopentyl]ethyl] thio]butoxy]benzoate. I.r. ($CHBr_3$) 1740, 1717cm$^{-1}$.

From Intermediate 15b.

16c) [1R-[1α, 2β(2R*), 3α]]-Methyl 4-[[2-[5-oxo-2-[3-phenoxy-2-[(tetrahydro-2H-pyran-2-yl)oxy]propoxy]-3-( etrahydro-2H-pyran-2-yl)oxy]cyclopentyl]ethyl]thio]buta noate. T.l.c. (ER-PE, 2:1) Rf 0.28.

From Intermediate 4.

16d) [1R-[1α, 2β(2R*), 3α]]-Methyl 4-[[2-[5-oxo-2-[3-phenoxy-2-[[tetrahydro-2H-pyran-2-yl)oxy]propoxy]-3-[ etrahydro-2H-pyran-2-yl)oxy]cyclopentyl]ethyl]sulfinyl] butanoate. I.r. ($CHBr_3$) 1738cm$^{-1}$.

From Intermediate 17a.

The following compounds were similarly prepared except that purification by chromatography was effected using 2:1 ER-PE→ER as eluant.

16e) [1R-[1α, 2β(2R*), 3α]]-Methyl 3-[[2-[5-oxo-2-[3-phenoxy-2-[(tetrahydro-2H-pyran-2-yl)oxy]propoxy]-3-[ tetrahydro-2H-pyran-2-yl)oxy]cyclopentyl]ethyl]thio]pro pionate as an oil (0.09g). T.l.c. (ER-PE, 2:1) Rf 0.24.

From Intermediate 13a (0.44g).

16f) [1R-[1α, 2β(2R*), 3α]]-Methyl 5-[[[5-oxo-2-[3-phenoxy-2-[(tetrahydro-2H-pyran-2-yl)oxy]propoxy]-3-[(t trahydro-2H-pyran-2-yl)oxy]cyclopentyl]methyl]thio]pent anoate. I.r. ($CHBr_3$) 1 740cm$^{-1}$.

From Intermediate 13b.

16g) [1R-[1α, 2β(2R*), 3α]]-Methyl [[3-[5-oxo-2-[3-phenoxy-2-[(tetrahydro-2H-pyran-2-yl)oxy]propoxy]-3-[(t trahydro-2H-pyran-2-yl)oxy]cyclopentyl]propyl]thio]acet ate. I.r. ($CHBr_3$) 1735cm$^{-1}$.

From Intermediate 13c.

16h) [1R-[1α, 2β(2R*), 3α]]-Methyl 3-[[3-[5-oxo-2-[3-phenoxy-2-[(tetrahydro-2H-pyran-2-yl)oxy]propoxy]-3-[ tetrahydro-2H-pyran-2-yl)oxy]cyclopentyl]propyl]thio]pr opionate. I.r. ($CHBr_3$) 1735cm$^{-1}$.

From Intermediate 13d.

16i) [1R-[1α, 2β(2R*), 3α]]-(-)-Methyl [[4-[5-oxo-2-[3-phenoxy-2-[(tetrahydro-2H-pyran-2-yl)oxy]propoxy]-3-[(t trahydro-2H-pyran-2-yl)oxy]cyclopentyl]butyl]thio]aceta te. I.r. ($CHBr_3$) 1736cm$^{-1}$. $[\alpha]_D^{20}$ − 15.3° ($CHCl_3$).

From Intermediate 13e.

16j) [1R-[1α, 2β(2R*), 3α]]-Methyl [[4-[5-oxo-2-[3-phenoxy-2-[(tetrahydro-2H-pyran-2-yl)oxy]propoxy]-3-[(t trahydro-2H-pyran-2-yl)oxy]cyclopentyl]butyl]sulfinyl]a cetate. I.r. ($CHBr_3$) 1732cm$^{-1}$.

From Intermediate 17b.

16k) [1R-[1α, 2β(2R*), 3α]]-Methyl [[4-[5-oxo-2-[3-phenoxy-2-[(tetrahydro-2H-pyran-2-yl)oxy]propoxy]-3-(te rahydro-2H-pyran-2-yl)oxy]cyclopentyl]butyl]sulfonyl]ac etate. I.r. ($CHBr_3$) 1740cm$^{-1}$.

From Intermediate 17c.

Intermediate 17

17a) [1S-[1α, 2β(2S*), 3α, 5α]]-Methyl 4-[[2-[5-hydroxy-2-[3-phenoxy-2-[(tetrahydro-2H-pyran-2-yl)oxy]propoxy] 3-[(tetrahydro-2H-pyran-2-yl)oxy]cyclopentyl]ethyl]sulf inyl] butanoate.

A solution of sodium periodate (0.25g) in water (5mℓ) was added to a stirred solution of Intermediate 4 (0.22g) in MeOH (10mℓ). After 18h EA (50mℓ) was added and the solution washed with half-saturated brine (40mℓ) and saturated brine (40mℓ). The washings were back-extracted with EA (40mℓ) and the combined organic layers dried. Evaporation and purification by chromatography using EA-MeOH (92:8 → 85:15) as eluant gave the titlecompound as an oil (0.18g). I.r. ($CHBr_3$) 3520, 1730cm$^{-1}$.

The following compound was prepared in a similar manner except that purification by chromatography was effected using EA-MeOH (16:1) as eluant .

17b) [1S-[1α, 2β(2S*), 3α, 5α]]-(+)-Methyl [[4-[5-hydroxy-2-[3-phenoxy-2-[(tetrahydro-2H-pyran-2-yl)oxy]propoxy]-3 [(tetrahydro-2H-pyran-2-yl)oxy]cyclopentyl]butyl]sulfin yl]acetate as an oil (0.39g). I.r. ($CHBr_3$) 3520, 1733cm$^{-1}$. $[\alpha]_D^{20}$ + 24$^0$ ($CHCl_3$).

From Intermediate 13e (0.39g).

17c) [1S-[1α, 2β(2S*), 3α, 5α]]-Methyl [[4-[5-hydroxy-2-[3-phenoxy-2-[(tetrahydro-2H-pyran-2-yl)oxy]propoxy]-3 [(tetrahydro-2H-pyran-2-yl)oxy]cyclopentyl]butyl]sulfon yl]acetate

$NaHCO_3$ (0.2g) and meta-chloroperbenzoic acid (80-90% pure, 0.07g) were added to a stirred of Intermediate 17b (0.2g) in $CH_2Cl_2$ (5mℓ) at 0°c. After 1h the solution was allowed to warm slowly to room temperature and, after a further hour, further meta-chloroperbenzoic acid (5mg) was added. After a further 2h ER (40mℓ) was added and the solution washed with 8% aqueous $NaHCO_3$ (40mℓ) and saturated brine (40mℓ). The aqueous washings were back-extracted with ER (40mℓ) and the combined organic layers dried. Solvent evaporation gave the titlecompound as an oil (0.19g). I.r. ($CHBr_3$), 3590, 3520, 1740cm$^{-1}$.

Example 1

[1R-[1α, 2β(R*), 3α]]4-(Benzoylamino)phenyl 4-[[2-[3-hydroxy-2-(2-hydroxy-3-phenoxypropoxy)-5-oxocyclopentyl]ethyl] hio]butanoate

A solution of Intermediate 16a (0.24g) in acetic acid-water-THF (20:10:3, 7mℓ) was heated at 50° for 4.5h. The solvent was removed invacuo and the residue purified by chromatography on acid-washed (pH 3.8) silica using EA - cyclohexane (3:1) as eluant to give the titlecompound as a white solid (0.09g), m.p. 103-5°.

Analysis Found : C,65.28; H,5.98; N,2.18.

$C_{33}H_{37}NO_8S$ requires C,65.22; H,6.14; N,2.30%.

The following compounds were prepared in a similar manner.

Example 2

[1R-[1α, 2β(R*), 3α]]-Methyl 4-[[4-[2-[3-hydroxy-2-(2-hydroxy-3-phenoxypropoxy)-5-oxo-cyclopentyl]eth l]thio]-1-oxobutoxy]benzoate as an oil (0.12g). I.r. ($CHBr_3$) 3580, 3440, 1745, 1718cm$^{-1}$.

Analysis Found : C,61.64; H,6.28.

$C_{28}H_{34}O_9S$ requires : C,61.6; H,6.23%.

From Intermediate 16b (0.3g) except that toluene-acetonitrile (2:1) was used as eluant.

Example 3

[1R-[1α, 2β(R*), 3α]]-(-)-Methyl 4-[[2-[3-hydroxy-2-(2-hydroxy-3-phenoxypropoxy)-5-oxocyclopentyl]ethyl] hio]butanoate as an oil (0.2g).

I.r. ($CHBr_3$) 3580, 3450, 1738cm$^{-1}$. $[\alpha]_D^{20}$ – 36.0$^0$ ($CHCl_3$).

Analysis Found : C,58.87; H,7.19.

$C_{21}H_{30}O_7S$ requires C,59.13; H,7.09%.

From Intermediate 16c (0.4g) except that ER-MeOH (97:3) was used as eluant.

Example 4

[1R-[1α, 2β(R*), 3α]]-Methyl 4-[[2-[3-hydroxy-2-(2-hydroxy-3-phenoxypropoxy)-5-oxocyclopentyl]ethyl] hio]sulfinyl]butanoate as an oil (0.066g).
T.l.c. (EA-MeOH, 9:1) Rf 0.29. I.r. ($CHBr_3$) 3580, 1736cm$^{-1}$.

From Intermediate 16d (0.12g) except that EA-MeOH (9:1) was used as eluant.

Example 5

[1R-[1α, 2β(R*), 3α]]-Methyl 3-[[2-[3-hydroxy-2-(2-hydroxy-3-phenoxypropoxy)-5-oxocyclopentyl]ethyl]thio]propionate as an oil (0.03g).
T.l.c. (ER-MeOH, 97:3) Rf 0.23. I.r. ($CHBr_3$) 3580, 3440, 1735cm$^{-1}$.

From Intermediate 16e (0.09g) except that ER-MeOH (97:3) was used as eluant.

Example 6

[1R-[1α, 2β(R*), 3α]]-Methyl 5-[[[3-hydroxy-2-(2-hydroxy-3-phenoxy-propoxy)-5-oxocyclopentyl] methyl]thio]pentanoate as an oil (0.036g).
I.r. ($CHBr_3$) 3580, 3450, 1740cm$^{-1}$.
Analysis Found : C,59.06; H,7.16.
$C_{21}H_{30}O_7S$ requires C,59.13; H,7.09%.

From Intermediate 16f (0.12g) except that ER-MeOH (97:3) was used as eluant.

Example 7

[1R-[1α, 2β(R*),3α]]-Methyl [[3-[3-hydroxy-2-(2-hydroxy-3-phenoxypropoxy)-5-oxocyclopentyl]propyl]thio]acetate as an oil (0.077g).
I.r. ($CHBr_3$) 3530, 3440, 1737cm$^{-1}$.
Analysis Found : C,58.58; H,7.15.
$C_{20}H_{28}O_7S$ requires C,58.23; H,6.84%

From Intermediate 16g (0.18g) except that ER-MeOH (97:3) was used as eluent.

Example 8

[1R-[1α, 2β(R*), 3α]]-Methyl 3-[[3-[3-hydroxy-2-(2-hydroxy-3-phenoxypropoxy)-5-oxocyclopentyl]propyl thio]propionate as an oil (0.04g).
T.l.c. (ER-MeOH, 97:3) Rf 0.27. I.r. ($CHBr_3$) 3580, 3440, 1738, 1734cm$^{-1}$.

From Intermediate 16h (0.093g) except that ER-MeOH (97:3) was used as eluant.

Example 9

[1R-[1α, 2β(R*), 3α]]-(-)-Methyl [[4-[3-hydroxy-2-(2-hydroxy-3-phenoxypropoxy)-5-oxocyclopentyl]butyl]thio]acetate as an oil (0.1g).
I.r. ($CHBr_3$) 3590, 3450, 1740cm$^{-1}$. $[\alpha]_D^{20}$ – 40.6° ($CHCl_3$).
Analysis Found : C,58.98; H,7.33.
$C_{21}H_{30}O_7S$ requires C,59.13; H,7.09%.

From Intermediate 16i (0.2g) except that ER-MeOH (97:3) was used as eluant.

Example 10

[1R-[1α, 2β(R*), 3α]]-Methyl [[4-[3-hydroxy-2-(2-hydroxy-3-phenoxypropoxy)-5-oxocyclopentyl]butyl]sulfinyl]acetate as an oil (0.068g).
I.r. ($CHBr_3$) 3590, 1740cm$^{-1}$.
Analysis Found : C,57.12; H,7.09.
$C_{21}H_{30}O_8S$ requires C,57.00; H,6.83%.

From Intermediate 16j (0.12g) except that ER-MeOH (97:3) was used as eluant.

Example 11

[1R-[1α, 2β(R*), 3α]]-Methyl [[4-[3-hydroxy-2-(2-hydroxy-3-phenoxypropoxy)-5-oxocyclopentyl]butyl]sulfonyl]acetate as an oil (0.084g).
T.l.c. (ER-MeOH, 97:3) Rf 0.27. I.r. ($CHBr_3$) 3590, 3450, 1742 cm$^{-1}$.

From Intermediate 16k (0.14g) except that ER-MeOH (97:3) was used as eluant.

The following are examples of pharmaceutical formulations using compounds of the invention. In the examples, the term "active ingredient" is used to denote a compound of the invention, such as a compound described in the preceding examples.

1. Tablets

These may be prepared by direct compression

| 1. Tablets | |
|---|---|
| These may be prepared by direct compression | |
| | mg/tablet |
| Active Ingredient | 0.015 to 0.2 |
| Magnesium stearate, BP | 1.5 |
| Microcrystalline cellulose, USP to compression weight | 150.0 |

The active ingredient is blended with about 10% of the microcrystalline cellulose then blended with the remaining microcrystalline cellulose and magnesium stearate. The blend is then compressed using 6mm diameter punches into tablets on a suitable machine.

The tablets may be film coated with suitable film forming materials e.g. methyl cellulose or hydroxypropyl methylcellulose using standard techniques.

2. Capsules

| 2. Capsules | |
|---|---|
| | mg/tablet |
| Active Ingredient | 0.015 to 0.2 |
| Magnesium stearate, BP | 1.0 |
| *Starch 1500 to fill weight | 150.0 |
| *A form of directly compression starch. | |

The active ingredient is preblended with some of the Starch 1500 then this preblend is mixed with the remaining Starch 1500 and magnesium stearate. The mix is then filled into size No 2 hard gelatin capsule shells using suitable machinery.

**Claims**

1. Compounds of the general formula (1)

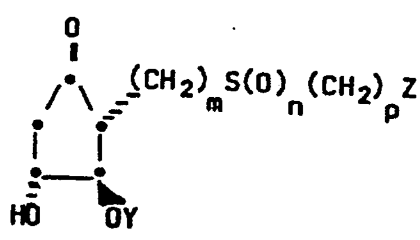

(1)

wherein
m is 1 to 4;
n is zero, 1 or 2;
p is 1 to 4;
Z is a group $CO_2R^1$ where $R^1$ is
(a) a hydrogen atom or a $C_{1-6}$ alkyl, $C_{7-10}$ phenalkyl or 2-naphthyl group;
(b) phenyl [optionally substituted by $C_{1-4}$ alkyl, $C_{1-4}$ alkoxy, $C_{1-4}$ alkanoyl, methylthio, methylsulphinyl,

methylsulphonyl, halogen, $-CO_2R^2$ (where $R^2$ is a hydrogen atom or $C_{1-4}$ alkyl or phenyl), $-NHCOR^2$ (where $R^2$ is as defined above or is a phenyl group optionally substituted by hydroxyl, $CH_3CONH-$ or

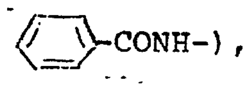,

$-CONR^3R^4$ (where $R^3$ and $R^4$ may be the same or different and are each a hydrogen atom or a $C_{1-4}$ alkyl group), $-NHCONH_2$, $-CH_2CH(CONH_2)NHCOCH_3$ or

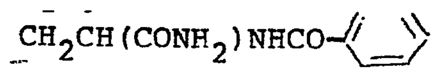 ];

(c) a group $-CH_2COR^5$ where $R^5$ is phenyl [optionally substituted by $C_{1-4}$ alkyl, $C_{1-4}$ alkoxy, $C_{1-4}$ alkanoyl, methylthio, methylsulphinyl, methylsulphonyl, halogen, $-CO_2R^6$ (where $R^6$ is a hydrogen atom or $C_{1-4}$ alkyl or phenyl), $-CONR^7R^8$ (where $R^7$ and $R^8$ may be the same or different and are each a hydrogen atom or a $C_{1-4}$ alkyl group), $-NHCOR^6$ (where $R^6$ is as defined above, or is a phenyl group optionally substituted by hydroxyl, $CH_3CONH-$ or

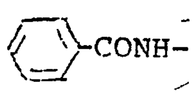

$-NHCONH_2$, $-CH_2CH(CONH_2)NHCOCH_3$ or

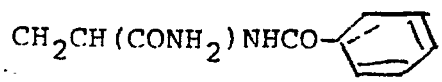 ];

or $R^5$ is 2-naphthyl;
or Z is $-CH_2OH$, $-CHO$ or $CONHR^9$ [where $R^9$ is a hydrogen atom or $C_{1-4}$ alkyl, aryl, $-COR^{10}$ (where $R^{10}$ is a hydrogen atom or a $C_{1-4}$ alkyl or aryl group) or $-SO_2R^{11}$ (where $R^{11}$ is a $C_{1-4}$ alkyl or aryl group)];

Y is 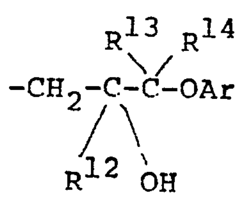

where $R^{12}$, $R^{13}$ and $R^{14}$ are each a hydrogen atom or a methyl and at least one is a hydrogen atom, and Ar is a phenyl group (optionally substituted by one or two $C_{1-4}$ alkyl, $C_{1-4}$ alkoxy, $C_{1-4}$ alkylthio, $C_{1-4}$alkylsulphinyl, $C_{1-4}$ alkylsulphonyl, halogen or trifluoromethyl groups);
and the physiologically acceptable salts, solvates and complexes thereof.

2. Compounds as claimed in claim 1 in which Z is a group $-COOR^1$ where $R^1$ is:
(a) a hydrogen atom, $C_{1-3}$ alkyl or 2-naphthyl group;
(b) phenyl substituted by a methoxy, acetyl, $-CO_2CH_3$, $-NHCOCH_3$, benzoylamino, $-CONH_2$, $-CON(CH_3)_2$ or $-CH_2CH(CONH_2)NHCOCH_3$ group;
(c) $-CH_2COR^5$ where $R^5$ is phenyl group substituted by a methoxy, acetyl, $-CO_2CH_3$, $-NHCOCH_3$, benzoylamino, $-CONH_2$, $-CON(CH_3)_2$ or $-CH_2CH(CONH_2)NHCOCH_3$ group,or $R^5$ is a 2-naphthyl group;
or Z is $-CH_2OH$, $-CHO$, $-CONH_2$, $-CONHCH_3$, $-CONHCOCH_3$, $-CONHSO_2CH_3$ or

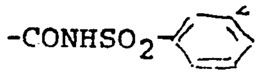 .

3. Compounds as claimed in claim 1 or claim 2 in which n is 0.

4. Compounds as claimed in any preceding claim in which $R^{13}$ and $R^{14}$ are hydrogen atoms and Ar is phenyl or phenyl substituted by chloro or fluoro.

5. Compounds as claimed in claim 1 in which:
n is zero
m is 3 or 4 and p is 1 or
m is 2 or 3 and p is 2 or
m is 1 and p is 4 or
m is 2 and p is 3;
Z is a group $CO_2R^1$ [where $R^1$ is a hydrogen atom of $C_{1-3}$ alkyl, 2-naphthyl or phenyl substituted by methoxy, acetyl, $-CO_2CH_3$, $-NHCOCH_3$, benzoylamino, $-CONH_2$, $-CON(CH_3)_2$ or $-CH_2CH(CONH_2)NHCOCH_3$];
$R^{12}$ is a hydrogen atom or methyl;
$R^{13}$ and $R^{14}$ are hydrogen atoms;
Ar is phenyl or phenyl substituted by fluoro or chloro;
and the physiologically acceptable salts, solvates and complexes thereof.

6. Compounds as claimed in claim 5 in which Z is a group $-CO_2R^1$ where $R^1$ is a hydrogen atom, methyl or phenyl substituted in the para-position by $-CO_2CH_3$ or benzoylamino.

7. Compounds as claimed in any preceding claim in which the carbon atom carrying the $-(CH_2)_mS(O)_n(CH_2)_pZ$group is in the R-configuration.

8. Compounds as claimed in claim 1, said compounds being:
[1 R-[1α,2β( R*), 3α]]-4-(benzoylamino)phenyl 4-[[2-[3-hydroxy-2-(2-hydroxy-3-phenoxypropoxy)-5-oxocyclopentyl]ethyl] hio]butanoate;
[1 R-[1α,2β( R*),3α]]-methyl 4-[[[[2-[3-hydroxy-2-(2-hydroxy-3-phenoxypropoxy)-5-oxocyclopentyl]ethy ]thio]-1-oxobutyl]oxy]benzoate; and
[1 R-[1α,2β( R*),3α]]-(-)methyl 4-[[2-[3-hydroxy-2-(2-hydroxy-3-phenoxypropoxy)-5-oxocyclopentyl]ethyl]thio]butanoate;
and complexes thereof.

9. A pharmaceutical composition comprising a compound as claimed in any preceding claim together with one or more pharmaceutical carriers.

10. A process for the preparation of a compound as claimed in claim 1 which comprises:
(a) deprotecting a compound of formula (2)

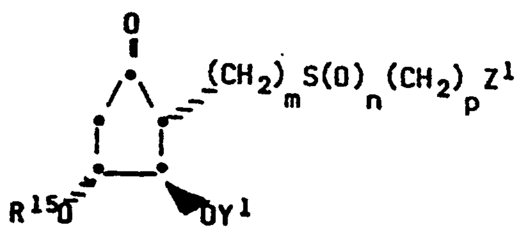

(2)

in which $Y^1$ is a group

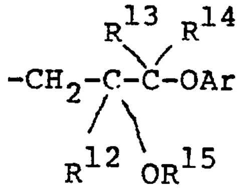

and $Z^1$ is Z as defined for formula (1) or is a group $-CH_2OR^{15}$ and $R^{15}$ is a hydroxyl protecting group;
(b) in the preparation of a compound in which Z is $-CO_2R^1$ where $R^1$ is a group of type (a) or (b), esterifying the corresponding compound in which Z is $-CO_2H$;
(c) in the preparation of a compound in which Z is $-CONHR^9$, amidating a corresponding compound of formula (1) in which Z is $-CO_2R^1$ (where $R^1$ is a hydrogen atom or a $C_{1-6}$ alkyl group);
(d) in the preparation of a compound in which n is 1, oxidising a corresponding compound in which n is 0;
(e) in the preparation of a compound in which n is 2, oxidising a corresponding compound in which n is 0 or 1;
(f) in the preparation of a compound in which Z is $-CO_2H$, hydrolysing a corresponding ester;
(g) in the preparation of a compound in which Z is $-CO_2R^1$ where $R^1$ is a group of type (c), alkylating the corresponding compound in which Z is $-CO_2H$ with a ketone of formula (12)
$XCH_2COR^5$(12)
in which x is a leaving group; or
(h) treating an acid of formula (1) with a base to form a salt or treating a compound of formula (1) with cyclodextrin to form a complex.

**Patentansprüche**

1. Verbindungen der allgemeinen Formel (1)

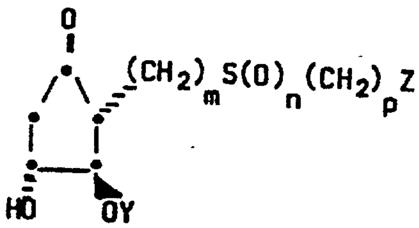

(1)

worin
m für 1 bis 4 steht;
n für 0,1 oder 2 steht;
p für 1 bis 4 steht;
Z eine Gruppe $CO_2R^1$ ist, worin $R^1$ bedeutet:
a) ein Wasserstoffatom oder eine $C_{1-6}$-Alkyl-, $C_{7-10}$Phenalkyl- oder 2-Naphthylgruppe;
b) Phenyl [gegebenenfalls substituiert durch $C_{1-4}$-Alkyl, $C_{1-4}$-Alkoxy, $C_{1-4}$-Alkanoyl, Methylthio, Methylsulfinyl, Methylsulfonyl, Halogen, $-CO_2R^2$ (worin $R^2$ für ein Wasserstoffatom oder für $C_{1-4}$-Alkyl oder Phenyl steht), $-NHCOR^2$ (worin $R^2$ wie vorstehend definiert ist oder eine Phenylgruppe bedeutet, die gegebenenfalls durch Hydroxyl, $CH_3CONH$- oder

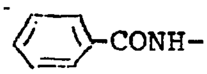 ) ,

substituiert ist),
$-CONR^3R^4$ (worin $R^3$ und $R^4$ gleich oder voneinander verschieden sein können und jeweils für ein Wasserstoffatom oder eine $C_{1-4}$-Alkylgruppe stehen), $NHCONH_2$, $-CH_2CH(CONH_2)NHCOCH_3$ oder

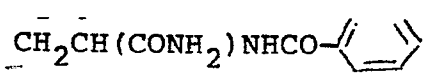 ];

c) eine Gruppe $-CH_2COR^5$, worin $R^5$ Phenyl [gegebenenfalls substituiert durch $C_{1-4}$-Alkyl, $C_{1-4}$-Alkoxy, $C_{1-4}$-Alkanoyl, Methyltio, Methylsulfinyl, Methylsulfonyl, Halogen, $-CO_2R^6$ (worin $R^6$ für ein Wasserstoffatom oder für $C_{1-4}$-Alkyl oder Phenyl steht), $-CONR^7R^8$ (worin $R^7$ und $R^8$ gleich oder voneinander verschieden sein können und jeweils für ein Wasserstoffatom oder eine $C_{1-4}$-Alkylgruppe stehen), $-NHCOR^6$ (worin $R^6$ wie vorstehend definiert ist oder eine Phenylgruppe bedeutet, die gegebenenfalls durch Hydroxyl, $CH_3CONH$- oder

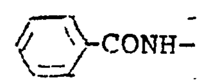

substituiert ist), $-NHCONH_2$, $-CH_2CH(CONH_2)NHCOCH_3$ oder

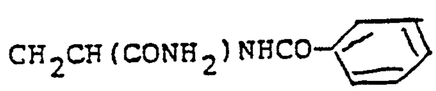 ]

bedeutet;
oder $R^5$ 2-Naphtyl ist;
oder Z $-CH_2OH$, $-CHO$ oder $CONHR^9$ [worin $R^9$ ein Wasserstoffatom oder $C_{1-4}$-Alkyl, Aryl, $-COR^{10}$ (worin $R^{10}$ für ein Wasserstoffatom oder für eine $C_{1-4}$-Alkylgruppe oder für eine Arylgruppe steht) oder $SO_2R^{11}$ (worin $R^{11}$ für eine $C_{1-4}$-Alkyl- oder eine Arylgruppe steht) bedeutet] bedeutet;

Y für 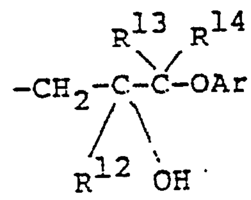

steht,
worin $R^{12}$, $R^{13}$ und $R^{14}$ jeweils ein Wasserstoffatom oder eine Methylgruppe sind, und zumindest einer dieser Reste ein Wasserstoffatom ist, und Ar für eine Phenylgruppe (gegebenenfalls substituiert durch ein oder zwei $C_{1-4}$-Alkyl-, $C_{1-4}$-Alkoxy-, $C_{1-4}$-Alkylthio-, $C_{1-4}$-Alkysulfinyl-, $C_{1-4}$-Alkylsulfonyl-, Halogen- oder Trifluormethylgruppen) steht;
und die physiologisch annehmbaren Salze, Solvate und Komplexe hiervon.

2. Verbindungen gemäß Anspruch 1, worin Z eine Gruppe $-COOR^1$ wiedergibt, worin $R^1$ bedeutet:
(a) ein Wasserstoffatom, eine $C_{1-3}$-Alkyl- oder 2-Naphthylgruppe;
(b) Phenyl, substituiert durch eine Methoxy-, Acetyl-, $-CO_2CH_3$-, $-NHCOCH_3$-, Benzoylamino-, $-CONH_2$-, $-CON(CH_3)_2$- oder $-CH_2CH(CONH_2)NHCOCH_3$-Gruppe;
(c) $-CH_2COR^5$, worin $R^5$ eine Phenylgruppe ist, die substituiert ist durch eine Methoxy-, Acetyl-, $-CO_2CH_3$-, $-NHCOCH_3$-, Benzoylamino-, $-CONH_2$-, $-CON(CH_3)_2$- oder $-CH_2CH(CONH_2)NHCOCH_3$-Gruppe
oder $R^5$ eine 2-Naphthylgruppe ist;
oder Z für $-CH_2OH$, $-CHO$, $-CONH_2$, $-CONHCH_3$, $-CONHCOCH_3$, $-CONHSO_2CH_3$ oder

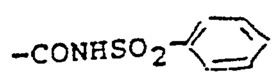

steht.

3. Verbindungen gemäß Anspruch 1 oder 2, worin n 0 ist.

4. Verbindungen gemäß einem der vorhergehenden Ansprüche, worin $R^{13}$ und $R^{14}$, Wasserstoffatome sind, und Ar Phenyl oder Phenyl, substituiert durch Chlor oder Fluor, bedeutet.

5. Verbindungen gemäß Anspruch 1, worin
n für 0 steht
m für 3 oder 4 steht und p 1 ist oder
m für 2 oder 3 steht und p 2 ist oder
m für 1 steht und p 4 ist oder
m für 2 steht und p 3 ist;
Z eine Gruppe $CO_2R^1$ [worin $R^1$ ein Wasserstoffatom oder $C_{1-3}$-Alkyl, 2-Naphthyl oder Phenyl, substituiert durch Methoxy, Acetyl, $-CO_2CH_3$, $-NHCOCH_3$, Benzoylamino, $-CONH_2$, $-CON(CH_3)_2$ oder $-CH_2CH(CONH_2)NHCOCH_3$, bedeutet] bedeutet;
$R^{12}$ für ein Wasserstoffatom oder Methyl steht;
$R^{13}$ und $R^{14}$ Wasserstoffatome sind;
Ar Phenyl oder Phenyl, substituiert durch Fluor oder Chlor, ist;
und deren physiologisch annehmbare Salze, Solvate und Komplexe.

6. Verbindungen gemäß Anspruch 5, worin Z eine Gruppe $-CO_2R^1$ ist, worin $R^1$ ein Wasserstoffatom, Methyl oder Phenyl, substituiert in para-Stellung durch $-CO_2CH_3$ oder Benzoylamino, ist.

7. Verbindungen gemäß einem der vorhergehenden Ansprüche, worin das Kohlenstoffatom, das die $-(CH_2)_mS(O)_n(CH_2)_pZ$-Gruppe trägt, sich in der R-Konfiguration befindet.

8. Als Verbindungen gemäß Anspruch 1 die folgenden Verbindungen:
[1R-[1α, 2β(R*), 3a]]-4-(Benzoylamino)-phenyl-4-[[2- [3-hydroxy-2-(2-hydroxy-3-phenoxypropoxy)-5-oxocyclopentyl]-ethyl]-thio]-butanoat;
[1R-[1α, 2β(R*), 3α]]-Methyl 4-[[[[2-[3-hydroxy-2-(2-hydroxy3-phenoxypropoxy)-5-oxocyclopentyl]-ethyl]-thio]-1oxobutyl]-oxy]-benzoat; und
[1R-[1α, 2β(R*), 3α]]-(-)Methyl-4-[[2-[3-hydroxy-2-(2-hydroxy-3-phenoxypropoxy)-5-oxocyclopentyl]-ethyl]-thio]butanoat;
und deren Komplexe.

9. Pharmazeutische Zusammensetzung, enthaltend eine Verbindung gemäß einem der vorhergehenden Ansprüche zusammen mit einem oder mehreren pharmazeutischen Trägern.

10. Verfahren zur Herstellung einer Verbindung gemäß Anspruch 1, das umfaßt
(a) die Schutzgruppenentfernung von einer Verbindung der Formel (2)

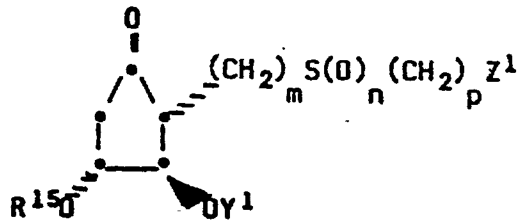

(2)

worin $Y^1$ für eine Gruppe

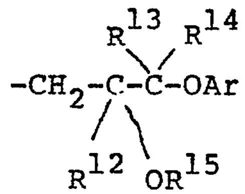

steht und $Z^1$ die Bedeutung von Z wie für Formel (1) definiert besitzt oder eine Gruppe $-CH_2OR^{15}$ ist, und $R^{15}$ eine Hydroxylschutzgruppe wiedergibt;
(b) bei der Herstellung einer Verbindung, worin Z $-CO_2R^1$ bedeutet, worin $R^1$ eine Gruppe des Typs (a) oder (b) ist, die Veresterung der entsprechenden Verbindung, worin Z für $-CO_2H$ steht:
(c) bei der Herstellung einer Verbindung, worin Z für eine Gruppe $-CONHR^9$ steht, die Amidierung einer entsprechenden Verbindung der Formel (1), worin Z für $-CO_2R^1$ steht (worin $R^1$ ein Wasserstoffatom oder eine $C_{1-6}$-Alkylgruppe bedeutet);
(d) bei der Herstellung einer Verbindung, worin n 1 ist, die Oxidation einer entsprechenden Verbindung, worin n 0 ist;
(e) bei der Herstellung einer Verbindung, worin n 2 ist, die Oxidation einer entsprechenden Verbindung, worin n 0 oder 1 ist;
(f) bei der Herstellung einer Verbindung, worin Z für $-CO_2H$ steht, die Hydrolyse eines entsprechenden Esters;
(g) bei der Herstellung einer Verbindung, worin Z für $-CO_2R^1$ steht, worin $R^1$ eine Gruppe des Typs (c) ist, die Alkylierung der entsprechenden Verbindung, worin Z für $-CO_2H$ steht mit einem Keton der Formel (12)

$XCH_2COR^5$

worin X für eine austretende Gruppe steht; oder
(h) die Behandlung einer Säure der Formel (1) mit einer Base, um ein Salz zu bilden oder die Behandlung einer Verbindung der Formel (1) mit Cyclodextrin, um einen Komplex zu bilden.

**Revendications**

1. Composés de formule générale (1)

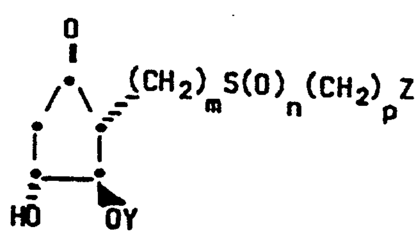

(1)

dans laquelle
m est 1 à 4;
n est zéro, 1 ou 2;
p est 1 à 4;
Z est un groupe $CO_2R^1$ dans lequel $R^1$ est

a) un atome d'hydrogène ou un groupe alcoyle en $C_1$ à $C_6$, un groupe phénylalcoyle en $C_7$ à $C_{10}$ ou un groupe 2-naphtyle;
(b) un phényle [éventuellement substitué par un alcanoyle en $C_1$ à $C_4$, par un alcoxy en $C_1$ à $C_4$, par un alcanoyle en $C_1$ à $C_4$, par un méthylthio, par un méthylsulfinyle, par un méthylsulfonyle, par un halogène, par $-CO_2R^2$ (dans lequel $R^2$ est un atome d'hydrogène ou un alcoyle en $C_1$ à $C_4$ ou un phényle), par $-NHCOR^2$ (dans lequel $R^2$ est tel que défini ci-dessus ou est un groupe phényle éventuellement substitué par hydroxyle, par $CH_3CONH-$ ou par

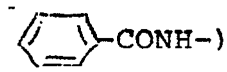 ) ,

par $-CONR^3R^4$ (dans lequel $R^3$ et $R^4$, qui peuvent être identiques ou différents, sont chacun un atome d'hydrogène ou un groupe alcoyle en $C_1$ à $C_4$), par $-NHCONH_2$, par $-CH_2CH(CONH_2)NHCOCH_3$ ou par $CH_2CH$ $(CONH_2)$

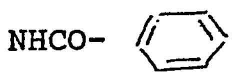 ];

c) un groupe $-CH_2COR^5$ dans lequel $R^5$ est phényle éventuellement substitué par un alcoyle en $C_1$ à $C_4$, par un alcoxy en $C_1$ à $C_4$, par un alcanoyle en $C_1$ à $C_4$, par un méthylthio, par un méthylsulfinyle, par un méthylsulfonyle, par un halogène, par $-CO_2R^6$ (dans lequel $R^6$ est un atome d'hydrogène ou un alcoyle en $C_1$ à $C_4$ ou un phényle), par $CONR^7R^8$ (dans lequel $R^7$ et $R^8$, qui peuvent être identiques ou différents, sont chacun un atome d'hydrogène ou un groupe alcoyle en $C_1$ à $C_4$), par $-NHCOR^6$ (dans lequel $R^6$ est tel que défini ci-dessus, ou est un groupe phényle éventuellement substitué par un hydroxyle, par $CH_3CONH-$ ou par

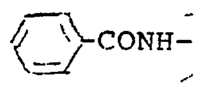

par $-NCONH_2$, par $-CH_2CH(CONH_2)NHCOCH_3$ ou par $CH_2CH(CONH_2)NHCO-$

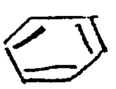 ]

ou $R^5$ est un 2-naphtyle;
ou Z est $-CH_2OH$, $-CHO$ ou $CONHR^9$ [dans lequel $R^9$ est un atome d'hydrogène ou un alcoyle en $C_1$ à $C_4$, un aryle, $-COR^{10}$ (dans lequel $R^{10}$ est un atome d'hydrogène ou un groupe alcoyle en $C_1$ à $C_4$ ou un groupe aryle) ou $-SO_2R^{11}$ (dans lequel $R^{11}$ est un groupe alcoyle en $C_1$ à $C_4$ ou un groupe aryle)];
Y est

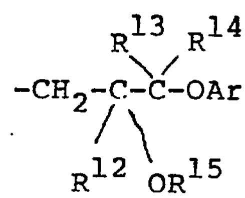

dans lequel $R^{12}$, $R^{13}$ et $R^{14}$ sont chacun un atome d'hydrogène ou un méthyle et l'un au moins d'entre eux est un atome d'hydrogène, et Ar est un groupe phényle (éventuellement substitué par un ou par deux groupes alcoyle en $C_1$ à $C_4$, alcoxy en $C_1$ à $C_4$, alcoylthio en $C_1$ à $C_4$, alcoylsulfinyle en $C_1$ à $C_4$, alcoylsulfonyle en $C_1$ à $C_4$, halogène ou trifluorométhyle);
et leurs sels, produits de solvatation et complexes physiologiquement acceptables.

2. Composés suivant la revendication 1, dans lesquels Z est un groupe $-COOR^1$ dans lequel $R^1$ est:
(a) un atome d'hydrogène, un groupe alcoyle en $C_1$ à $C_3$ ou un groupe 2-naphtyle;
(b) un phényle substitué par un groupe méthoxy, acétyle, $-CO_2CH_3$, $-NHCOCH_3$, benzoylamino, $-CONH_2$, $-CON(CH_3)_2$ ou $-CH_2CH(CONH_2)NHCOCH_3$;
(c) $-CH_2COR^5$ dans lequel $R^5$ est un groupe phényle substitué par un groupe méthoxy, acétyle, $-CO_2CH_3$, $-NHCOCH_3$, benzoylamino, $-CONH_2$, $-CON(CH_3)_2$ ou $-CH_2CH(CONH_2)NHCOCH_3$, ou $R^5$ est un groupe 2-naphtyle;

ou Z est $-CH_2OH$, $-CHO$, $-CONH_2$, $-CONHCH_3$, $-CONHCOCH_3$, ou $-CONHSO_2CH_3$ ou

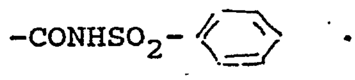

3. Composés suivant la revendication 1 ou 2, dans lesquels n est 0.

4. Composés suivant l'une des revendications précédentes, dans lesquels $R^{13}$ et $R^{14}$ sont des atomes d'hydrogène et Ar est phényle ou phényle substitué par un chlore ou par un fluor.

5. Composés suivant la revendication 1, dans lesquels:

n est zéro

m est 3 ou 4 et p est 1 ou

m est 2 ou 3 et p est 2 ou

m est 1 et p est 4 ou

m est 2 et p est 3;

Z est un groupe $CO_2R^1$ [dans lequel $R^1$ est un atome d'hydrogène ou un alcoyle en $C_1$ à $C_3$, un 2-naphtyle ou un phényle substitué par un méthoxy, par un acétyle, par $-CO_2CH_3$, par $-NHCOCH_3$, par un benzoylamino, par $-CONH_2$, par $-CON(CH_3)_2$ ou par $-CH_2CH(CONH_2)NHCOCH_3$];

$R^{12}$ est un atome d'hydrogène ou méthyle;

$R^{13}$ et $R^{14}$ sont des atomes d'hydrogène;

Ar est phényle ou phényle substitué par un fluor ou par un chlore;

et leurs sels, produits de solvatation et complexes physiologiquement acceptables.

6. Composés suivant la revendication 5, dans lesquels Z est un groupe $-CO_2R^1$ dans lequel $R^1$ est un atome d'hydrogène, un méthyle ou un phényle substitué en la position para par $-CO_2CH_3$ ou par un benzoylamino.

7. Composés suivant l'une des revendications précédentes, dans lesquels l'atome de carbone portant le groupe $(CH_2)_mS(O)_n(CH_2)_pZ$ est en la configuration R.

8. Composés suivant la revendication 1, qui sont:

le 4-[[2-[3-hydroxy-2-(2-hydroxy-3-phénoxypropoxy)-5-oxocyclopentyl]-éthyl]thio]butanoate de [1R-[1α,2β(R*),3α]]-4(benzoylamino)phényle;

le 4[[[[2-[3-hydroxy-2-(2-hydroxy-3-phénoxypropoxy)-5-oxocyclopentyl]éthyl]thio]-1-oxobutyl]oxy]benzoate de [1R-[1α,2β (R*), 3α]]-méthyle; et

le 4-[[2-[3-hydroxy-2-(2-hydroxy-3-phénoxypropoxy)-5-oxocyclopentyl]éthyl]thio]butanoate de [1R-[1α,2β(R*),3α]]-(-)méthyle;

et leurs complexes.

9. Composition pharmaceutique comprenant un composé tel que revendiqué à l'une des revendications précédentes, associé à un ou à plusieurs excipients pharmaceutiques.

10. Procédé de préparation d'un composé suivant la revendication 1, qui consiste:

a) à déprotéger un composé de formule (2)

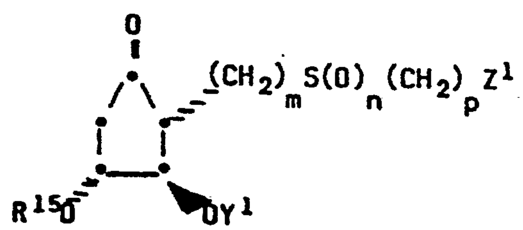

(2)

dans laquelle $Y^1$ est un groupe

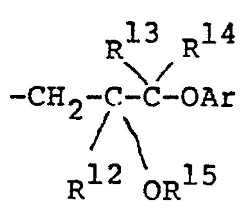

et $Z^1$ est Z tel que défini pour la formule (1) ou est un groupe $-CH_2OR^{15}$ et $R^{15}$ est un groupe protégeant un hydroxyle;

b) pour la préparation d'un composé dans lequel Z est $-CO_2 R^1$, $R^1$ étant un groupe du type (a) ou du type (b), à estérifier le composé correspondant dans lequel Z est $-CO_2H$;
c) pour la préparation d'un composé dans lequel Z est $CONHR^9$, à faire l'amide d'un composé correspondant de formule (1) dans lequel Z est $-CO_2R^1$ ($R^1$ étant un atome d'hydrogène ou un groupe alcoyle en $C_1$ à $C_6$);
d) pour la préparation d'un composé dans lequel n est 1, à oxyder un composé correspondant dans lequel n est 0;
(e) pour la préparation d'un composé dans lequel n est 2, à oxyder un composé correspondant dans lequel n est 0 ou 1;
(f) pour la préparation d'un composé dans lequel Z est $-CO_2H$, à hydrolyser un ester correspondant;
(g) pour la préparation d'un composé dans lequel Z est $CO_2R^1$, $R^1$ étant un groupe de type (c), à alcoyler le composé correspondant dans lequel Z est $-CO_2H$ par une cétone de formule (12)

$XCH_2COR^5$

dans laquelle X est un groupe labile; ou
h) à traiter un acide de formule (1) par une base pour former un sel ou à traiter un composé de formule (1) par la cyclodextrine pour former un complexe.